(19) Europäisches Patentamt
European Patent Office
Office européen des brevets

(11) Publication number: 0 342 035
A2

(12) # EUROPEAN PATENT APPLICATION

(21) Application number: 89304781.1

(22) Date of filing: 11.05.89

(51) Int. Cl.[4]: C 08 G 59/62
C 09 D 3/58, C 09 D 5/00

(30) Priority: 12.05.88 US 193498

(43) Date of publication of application:
15.11.89 Bulletin 89/46

(84) Designated Contracting States: DE FR GB IT

(71) Applicant: MINNESOTA MINING AND MANUFACTURING COMPANY
P.O. Box 33427
St. Paul Minnesota 55133-3427 (US)

(72) Inventor: Bymark, Richard M. c/o Minnesota Mining and Manufacturing Company 3M Austin Center
P.O. Box 2963 Austin Texas 78769-2963 (US)

Kirk, Alan R. c/o Minnesota Mining and Manufacturing Company 2501 Hudson Road P.O.Box 33427 St. Paul, Minnesota 55133-3427 (US)

Griggs, Allen L. c/o Minnesota Mining and Manufacturing Company 2501 Hudson Road P.O.Box 33427 St. Paul Minnesota 55133-3427 (US)

Martin, Steven J. c/o Minnesota Mining and Manufacturing Company 2501 Hudson Road P.O. Box 33427 St. Paul, Minnesota 55133-3427 (US)

(74) Representative: Baillie, Iain Cameron et al
c/o Ladas & Parry Isartorplatz 5
D-8000 München 2 (DE)

(54) **Powdered epoxy resin compositions.**

(57) A powdered coating composition contains an uncured epoxy resin having an epoxy equivalent weight greater than 99, and at least one aromatic compound having hydroxyl groups in adjacent or available adjacent positions. The coating composition is applied to a metal substrate, and is heated to coalesce and cure the composition into a tenacious, anti-corrosive coating.

EP 0 342 035 A2

**Description**

# POWDERED EPOXY RESIN COMPOSITIONS

## Background of the Invention

This application relates to powdered coating compositions containing uncured epoxy resins and aromatic compounds having hydroxy groups in adjacent or available adjacent positions. This application relates to methods for providing metal substrates with anti-corrosive coatings.

The coatings produced by the powdered coating compositions of this application demonstrate improved adhesive properties over coatings that do not contain the above-mentioned aromatic polyols. The coatings of this application demonstrate tenacious adherence to metal substrates under hot and wet conditions. Surface preparation of the metal substrates can be minimal. A tenacious coating is achieved even when the metal substrate is visibly oxidized.

U.S. Patent 4,560,732 discloses a coating composition that contains the reaction product of an epoxy resin, at least one polynuclear polyhydroxy phenol, and a solvent. This patent discloses working embodiments that contain a mixture of a diglycidyl ether of bisphenol A or F, a polynuclear polyhydroxy phenol compound, a solvent, and a catalyst. These mixtures are agitated and heated to produce a pre-cured coating composition. The pre-cured compositions are applied to metal substrates followed by solvent evaporation to yield anti-corrosive coatings.

The solvent vehicle required in the composition disclosed in U.S. Patent 4,560,732 limits the utility of the invention. One problem is attributable to the fact that the solvent must be removed as a final step in the coating process. Thus, a coating process which uses the composition of U.S. Patent 4,560,732 must provide means for handling and disposing of the solvent.

A second problem is complicated coating techniques. Solvent-applied coatings typically must be applied as a plurality of thin layers. The solvent-application technique is time and energy consuming, since each thin layer must be dried before a subsequent layer is applied.

## Summary of the Invention

An object of the present invention is to provide a curable powdered coating composition that demonstrates enhanced adherence and anti-corrosive properties when coated and cured on a metal substrate. The coating composition contains, in powdered form, a curable powdered coating composition comprising

(a) at least one uncured epoxy resin having an epoxy equivalent weight greater than 99, and

(b) one or more aromatic compounds containing at least one of the following moieties

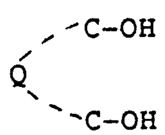

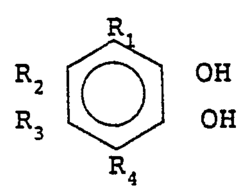

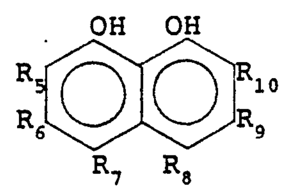

wherein Q comprises any combination of carbon, oxygen, nitrogen and/or sulfur atoms necessary to complete an aromatic ring and $R_1$-$R_{10}$ are independently a hydrogen or a substitution group. Adjacent substitution groups can form fused aromatic, nonaromatic or heterocyclic rings of up to 7 ring atoms.

The composition can further contain a curing agent and/or a curing catalyst.

The coating composition is applied in powder form to a metal substrate. The applied composition is then heated to coalesce and cure it into a corrosion-inhibiting coating.

The powdered coatings of this application exhibit improved adhesion under hot wet conditions, improved cathodic disbondment, and excellent adhesion to a substrate even when the coating is applied at lower than normal powder coating temperatures. Moreover, none of the problems associated with solvents, for example, pollution, fire hazard and toxicity, are observed. Thick coatings can be applied in a single application, unlike solvent-based coatings which are applied in multiple thin layers and require that each layer be dried in turn. Thus, production coating lines can operate at higher outputs.

## Detailed Description of the Invention

Suitable epoxy resins have an epoxy equivalent weight greater than 99. Preferably, the epoxy resins have an epoxy equivalent weight of 99 to 3500. More preferably, the epoxy resins have an epoxy equivalent weight of 550 to 1600. Suitable epoxy resins include the reaction products of a phenol compound having at least two hydroxy groups and an epihalogenohydrin, e.g., epichlorohydrin. These resins are commonly called bisphenol A and bisphenol F epoxy resins, and have the general formula

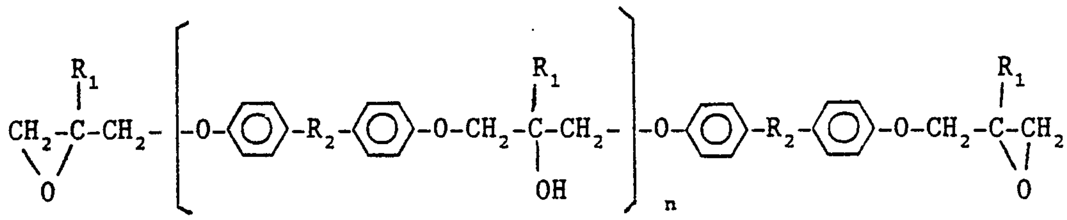

wherein $R_1$ is H or $CH_3$; $R_2$ is $-CH_2-$ or $-C(CH_3)_2-$; and n is a number from 2 to 25.

Another suitable epoxy resin is triglycidyl isocyanurate, which has the general formula

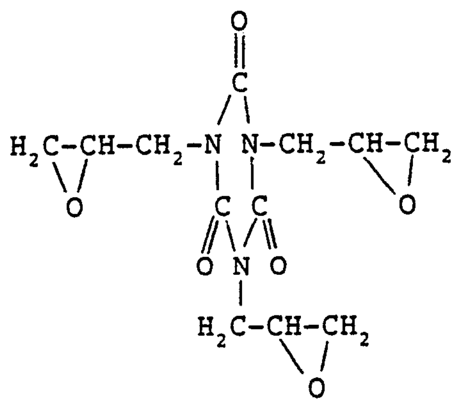

Other suitable epoxy resins are the novolak types having the general formula

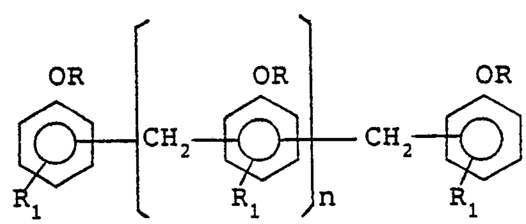

wherein R = H, 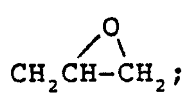; $R_1$ = H, $CH_3$; and n = 0-8.

Examples of commercially available resins which can be employed are Epon 2004 manufactured by Shell Oil Company, and DER 663 and 667 manufactured by Dow Chemical Company, Epi Rez 522, 530, 540 or SU 4, 5, 8 manufactured by Interez Inc. and PT-810 or ECN 1235, 1273, 1280, 1299 manufactured by Ciba-Geigy Corporation.

The aromatic compounds to be admixed with the epoxy resins contain moieties of the general formulae

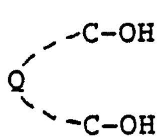 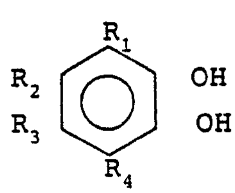 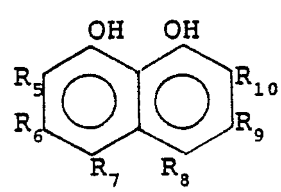

wherein Q comprises any combination of carbon, oxygen, nitrogen and/or sulfur atoms necessary to complete an aromatic ring and $R_1$-$R_{10}$ are independently a hydrogen or a substitution group. Adjacent substitution groups can form fused aromatic, nonaromatic or heterocyclic rings of up to 7 ring atoms. Suitable substitution groups may be any of the known aromatic ring substituents. Examples of suitable substitution groups include halogen, cyano, nitro, azo, nitroso, alkyl, aryl, alkoxy, aroyl, aryloxy, alkylthio, arylthio, alkyl(aryl)sulfinyl, alkyl(aryl)sulfonyl, formyl, amino, mercapto, hydroxy, carboxy, sulfo, carbamoyl, alkanoyl, alkenyl, and alkynyl. Hydrocarbon-containing substitution groups are preferabiy up to 18 carbon atoms in length. It is to be understood that the above-named groups can be either substituted or unsubstituted.

These compounds can generically be termed aromatic compounds having at least two hydroxyl groups on

adjacent or available adjacent positions. The aromatic compounds are based upon aromatic moieties such as phenyl, naphthyl, pyridyl or anthryl and the like. In the case of mononuclear aromatic moieties, the hydroxyls are on adjacent carbon atoms:

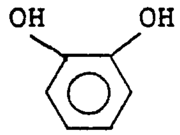

In the case of fused polynuclear aromatic moieties, the hydroxyls are on available adjacent positions:

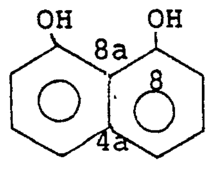

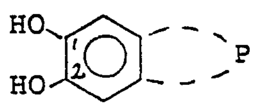

wherein P represents fused aromatic, nonaromatic or heterocyclic rings of up to 7 ring atoms. As indicated above, the 1 and 8 positions and the 1 and 2 positions are available adjacent positions, since the 4a and 8a positions cannot be substituted.

The aromatic compounds can be used by themselves or in derivative form. Examples of aromatic compounds having adjacent hydroxy groups are pyrogallol, catechol and 2,3-pyridine diol. Examples of the derivative forms are the condensation products of pyrogallol and catechol with formaldehyde, acetone or other ketones. Also, copolymerization of the above with other phenols which do not contain adjacent diols is acceptable. Other examples of the derivative forms are the carboxylic acids, esters, amides, alcohols and ketones of catechol and pyrogallol, e.g., 5,5′,6,6′-tetrahydroxy-3,3,3′,3′-tetramethylbis-1,1′-spirohydrindene, N-(4-Methylpiperizinyl)-3′,4′,5′-trihydroxybenzamide, 3,3′,4,4′,5-pentahydroxybenzophenone, catechol/phenol/formaldehyde novolak resin, catechol-formaldehyde novolak, and propylgallate. The condensation products of catechol with formaldehyde are preferred, since they are easily prepared as non-tacky powders.

The fused polynuclear aromatic compounds can be used by themselves or in derivative form. Examples of fused polynuclear aromatic polyols are dithranol, 1,8-dihydroxynaphthalene, 2,3-dihydroxynaphthalene and 1,2-dihydroxyanthraquinone. The derivative forms correspond to the carboxylic acid, ester, amide, alcohol, and ketone derivatives of the fused polyhydroxy phenols discussed above. The derivative forms further include polymers formed from repeating units of the fused polynuclear aromatic compounds.

The foregoing aromatic compounds are preferably present in the composition in amounts as low as 0.01 parts by weight per 100 parts of epoxy resin. More preferably, the amount of aromatic compound ranges from 0.05 to 100 parts by weight per 100 parts of epoxy resin.

The composition can also contain fillers, pigments, and flow control agents. Furthermore, although a mixture of the epoxy resin and aromatic compound is heat-curable by itself, it is preferable that the composition contain a catalyst and/or additional cure agents.

Suitable fillers are those known in the art, e.g. calcium metasilicate, ground silica, various micas, calcium carbonate, magnesium oxide, barium sulfate and the like. The fillers can be present in amounts up to about 40% by weight.

Suitable pigments are those known in the art, e.g., titanium dioxide, zinc oxide, and various iron oxide pigments. The pigments can be present in the coating composition in amounts up to about 40% by weight.

Suitable flow control agents are those known in the art, e.g. various polyacrylates, silicones and fluorocarbons. The flow control agents can be present in the coating composition in amounts up to about 2% by weight.

Suitable curing agents are those known in the art to cure epoxy resins, e.g., amine type curing agents such as dicyandiamide, methylene dianiline and dihydrazides, as well as anhydride type curing agents such as benzophenone tetracarbosylic dianhydride (BTDA), carboxyl terminated polyesters, and novolak resins and the like. The during agents can be present in the coating composition in amounts which depend on the curing stoichiometry. Generally, the amount of curing agent in the composition will range between about 1 and 50% by weight.

Suitable catalysts are those known in the art to catalyze the cure of epoxy resins, e.g., 2-methylimidazole adducts, 2,4,6-tris(dimethylaminomethyl)phenol, N,N-dimethylbenzylamine and stannous octoate. The catalysts can be present in the coating composition in amounts up to about 5% by weight.

The coating composition is formulated to remain as a free-flowing powder at ambient conditions. The powder coating composition can be applied to a substrate in any of the conventional powder coating techniques. Suitable techniques are fluid bed and spray coating, with or without electrostatic techniques.

Suitable temperatures are employed to melt, coalesce and cure the powder coatings. Heating may be effected by any suitable method such as convection or direct impingement ovens, IR or induction.

Preparation of Catechol/Formaldehyde Condensation Products

440.4 grams of catechol (4.0 moles) and 162 grams of 37% aqueous formaldehyde (2.0 moles) are placed into a one-liter, three-necked, round-bottomed flask equipped with a paddle stirrer, thermometer, water-cooled condenser and heating mantle. 400 milliliters of deionized water are added and stirring begins. The mixture is heated to 50°C, 75°C, 85°C, and finally to reflux, over 15-minute intervals. Reflux is continued for two hours total, after which the solution is cooled to about 60°C. 4 grams (0.044 moles) of oxalic acid (catalyst) are added and the temperature is raised to reflux over a 30-minute period. Reflux is continued, totaling an additional two hours. The heat of reaction (exotherm) is easily controlled by this procedure. At the end of the reaction period, 50 millimeters Hg vacuum is applied. The pressure is gradually lowered to less than or equal to 0.8 millimeters Hg vacuum and the temperature of the mixtures allowed to rise between 150 and 160°C. The mixture is maintained at this temperature and vacuum for 30 minutes. The vacuum and heat are removed and the product is poured into an aluminum pan. When cooled to room temperature, the reddish product is broken into smaller pieces and stored in an air-tight container. Yield: 450 grams.

The product is analyzed by gel permeation chromatography and determined to have a number average molecular weight of 365, a weight average molecular weight of 855, and a Z average molecular weight of 1522. The polydispersity is 2.34. Differential scanning calorimetry analysis reveals a midpoint glass transition temperature of 59°C. High performance liquid chromatography, using a reverse phase column, reveals the product to contain 20% free catechol. Fourier transform infrared spectroscopy reveals a characteristic phenol hydroxyl group at 3375 5 $cm^{-1}$, and other characteristic bands at 1515, 1282, 1258 and 1190 $cm^{-1}$.

Vacuum Distillation of Calechol/Formaldehyde Condensation Products

The product obtained above is distilled under reduced pressure (0.3 to 0.8 mm Hg) while heating at 200°C + 5°C and held at these conditions for one hour. The product is cooled to room temperature..

Preparation of 3,3′,4,4′,5-Pentahydroxybenzophenone

20.0 g (0.10 moles) of freshly distilled 3,4-dimethoxy-benzoyl chloride are added to a 1 liter flask equipped with heating mantle, stirrer, thermometer and water cooled condenser. To this flask is added 100 mls of 1,2-dichloroethane. The stirring is begun as 14.7 g (0.11 moles) of anhydrous $AlCl_3$ are added in three portions. After stirring the red complex for 5 minutes, a solution of 17.0 g (0.101 moles) of 1,2,3-trimethoxybenzene in 100 ml of 1,2-dichloroethane is added dropwise to the reaction contents over 30 minutes. The contents are further reacted at 50°C for another hour and then cooled to room temperature. The reaction mixture is hydrolyzed with 10% hydrochloric acid. The organic layer is washed with water and evaporated to give a yellow oil. Trituration of this oil with hexane gives a pale green solid, mp. 117 20°C. The yield is 29 gms (87%). The product is 3,3′,4,4′,5,-pentamethoxybenzophenone, and is identified by infrared and NMR spectroscopy. In another reaction, a 0.30 mole scale yields 81% of the desired product.

50.0 g (0.15 moles) of the above pentamethoxybenzophenone are placed into a 500 ml flask. To the flask are added 116 g (1.0 moles) of pyridine hydrochloride and the reaction contents are brought to reflux for 1-1/2 hrs. The reaction contents are cooled, quenched with water and extracted with ethyl acetate. The organic solution is dried over a desiccating agent and concentrated. The product is triturated with hexane to give 33 g (84% yield) of 3,3′,4,4′,5-pentahydroxybenzophenone. The product is identified by IR, NMR and melting point.

Preparation of Pyrogallol/Acetone Condensation Products

225 g (1.78 moles) of pyrogallol are placed into a 2 liter round bottomed flask equipped with a paddle stirrer, condenser, thermometer and mantle. To this flask are added 360 ml of acetone and 300 ml of 1,2-dichloroethane (DCE). The mixture is stirred as 23 ml of $POCl_3$ are added dropwise. The temperature of the reaction rises from 25°C to 70°C, but is kept below 70°C with occasional cooling. When all of the POCls has been added, heat: is applied to maintain the temperature of the reaction at 70°C for 2 hours. The reaction is cautiously hydrolyzed by pouring into water. The organic products are extracted with ethyl acetate, washed again with water, dried over $MgSO_4$, filtered and stripped to dryness under vacuum. The recovered oil is dried at 65°C. The solid obtained has a brown color.

Preparation of Catechol/Acetone Condensation Products

5,5′,6,6′-Tetrahydroxy-3,3,3′,3′-tetramethylbis-1,1,-spirohydrindene is prepared according to the procedure of Baker, Journal of the Chemical Society, 1934, pp. 1678-1681. The product, when triturated in hot ethanol, is an off-white powder, mp > 300°C. The identity of the material is established by proton NMR and is further characterized by IR.

Preparation of Pyrogallol/Formaldehyde Condensation Products

504.4 g (4 moles) of pyrogallol, 350 ml of water and 162 g (2 moles) of 37% aqueous formaldehyde are placed into a 1 liter 3 necked flask equipped with a paddle stirrer, thermometer, condenser and mantle. The solution is stirred and heated to reflux for 2 hrs, and then cooled to room temperature. To this is added 4.0 g (44.4 moles) of anhydrous oxalic acid and the reaction brought to reflux for an additional 2 hours. The reaction mixture is dehydrated under vacuum. When the temperature of the resin reaches 150-160°C @ 1.0 mm Hg or less, it is drained and allowed to cool. The brittle, green, solid resin is broken into lumps and stored in an air-tight container. The yield is 450 gms.

Preparation of N-(4-MethY-ylpiperazinyl)-3',4',5,-trihydroxybenzamide

36.8 g (0.20 moles) of methyl gallate are dissolved with 250 ml of ethyl acetate in a 500 ml flask. To this is added, in one portion, 20.1 g (0.20 moles) of 1-methylpiperazine. The reaction is heated to reflux, then cooled. Ethyl-ethyl ether is added to the reaction mixture and the contents filtered. 36 g (95%) of a white powder is obtained, mp 135-138°C. The material is identified by IR, NMR and melting point.

Preparation of Powdered Coating Compositions

Formulations are prepared on a rubber mill (cold roll cooled by cold tap water, hot roll heated to about 65°C). All components except curing agents and catalysts are added and kneaded for 4-5 minutes. Subsequent addition of curing agent and catalyst is followed by 5 additional minutes of banding and kneading. The resin is ground to a fine powder with a hammer mill and screened through a 212 µm mesh.

Coating Technique

Steel bars measuring 1" x 6-7/8" x 3/8" (blasted with steel shot) are heated to the specified temperature, dipped into a fluidized bed of a powdered coating composition to a cured coating thickness of 350-410 µm, and allowed to air cool to room temperature.

The following compositions are prepared and subsequently coated on steel bars.

| Example | 1 | 2 | 3 | 4 | 5 | 6 | 7 | 8 |
|---|---|---|---|---|---|---|---|---|
| 2004 | 200 | 200 | 200 | 200 | | | 200 | 200 |
| 663 u | | | | | 200 | | | |
| 667 | | | | | | 200 | | |
| P-1 | 70 | 70 | 70 | 70 | 70 | 70 | 70 | 70 |
| $TiO_2$ | 10 | 10 | 10 | 10 | 10 | 10 | 10 | 10 |
| MFP | 2 | 2 | 2 | 2 | 2 | 2 | 2 | 2 |
| dicy | 3.75 | 3.75 | 3.75 | 3.75 | 4.5 | 2.0 | 3.75 | 3.75 |
| 2-MI | 1 | 1 | 1 | 1 | 1.2 | 0.5 | 1 | 1 |
| DMP-30 | 3 | 3 | 3 | 3 | 3.6 | 1.6 | 3 | 3 |
| a | | 4 | | | | | | |
| b | | | 4 | | | | | |
| c | | | | 4 | | | | |
| d | | | | | 4 | | | |
| e | | | | | | 4 | | |
| f | | | | | | | 4 | |
| g | | | | | | | | 4 |

| Example | 9 | 10 | 11 | 12 | 13 | 14 | 15 | 16 |
|---|---|---|---|---|---|---|---|---|
| 2004 | 200 | 200 | 200 | 200 | 200 | 200 | 200 | 200 |
| P-1 | 70 | 70 | 70 | 70 | 70 | 70 | 70 | 70 |
| $TiO_2$ | 10 | 10 | 10 | 10 | 10 | 10 | 10 | 10 |
| $Cr_2O_3$ | | | | | | | | |
| MFP | 2 | 2 | 2 | 2 | 2 | 2 | 2 | 2 |
| dicy | | | | | 3 | 3 | 3 | 3 |
| 2-MI | 1 | 1 | 1 | 1 | 1 | 1 | 1 | 1 |
| 417 | 24 | 24 | | | | | | |
| TMA | | | 20 | 20 | | | | |
| h | | 4 | | | | | | |
| i | | | | 4 | 0.2 | 0.4 | 1.0 | 2.0 |
| j | | | | | | | | |
| k | | | | | | | | |
| l | | | | | | | | |
| m | | | | | | | | |

| Example | 17 | 18 | 19 | 20 | 21 |
|---|---|---|---|---|---|
| 2004 | 200 | 200 | 200 | 200 | 200 |
| P-1 | 70 | 70 | 88 | 88 | 88 |
| $TiO_2$ | 10 | 10 | 1.7 | 1.7 | 1.7 |
| $Cr_2O_3$ | | | 5.0 | 5 | 5 |
| MFP | 2 | 2 | 2 | 2 | 2 |
| dicy | 3 | 3 | 3 | 3 | 3 |
| 2-MI | 1 | 1 | 1 | 1 | 1 |
| 417 | | | | | |
| TMA | | | | | |
| h | | | | | |
| i | | | | | |
| j | | 1 | | | |
| k | | | 2 | | |
| l | | | | 2 | |
| m | | | | | 2 |

| Example | 22 | 23 |
|---|---|---|
| Arakote 3003 | 186 | 186 |
| P-1 | 84 | 84 |
| $TiO_2$ | 7 | 7 |
| MFP | 2 | 2 |
| PT-810 | 14 | 14 |
| Veba B-31 | 1.4 | 1.4 |
| i | | 4 |

all numbers are parts by weight

2004 = Shell 2004 epoxy resin E.E.W. 875-975
663u = Dow D.E.R. 663 epoxy resin E.E.W. 730-840
667 = Dow D.E.R. 667 epoxy resin E.E.W. 1,600-2,000
P-1 = NYEO calcium metasilicate
MFP = Synthron proprietary acrylic polymer absorbed on micronized silica (flow control agent)
dicy = dicyandiamide
2-MI = 2-Methylimidazole
DMP-30 = Pacific Anchor 2,4,6-tris(dimethylaminomethyl)-phenol
417 = Shell RSC -417 phenolic E.W. 155
TMA = trimellitic anhydride
Arakote 3003 = Ciba-Geigy carboxyl terminated polyester resin E.W. 1889
PT-810 = Ciba-Geigy triglycidyl isocyanurate
Veba B-31 = imidazoline
a = 5,5',6,6'-tetrahydroxy-3,3,3',3'-tetramethylbis-1,1'-spirohydrindene
b = 3,3',4,4',5-pentahydroxybenzophenone
c = N-(1-methylhexyl)-3',4',5'-trihydroxybenzamide
d = t-butylcatechol/formaldehyde novolak 0.67 HCHO/TBC
e = catechol
f = 1,3,5-trihydroxybenzene
g = 4'-amino-3,4-dihydroxybenzophenone

h = catechol/phenol/formaldehyde novolak 50:50 cat./phenol 0.67 HCHO/phenols

i = pyrogallol

j = dithranol

k = 1,8-dihydroxynaphthalene

l = 1,2-dihydroxyanthraquinone

m = 2,3-dihydroxypyridine

Hot Water Immersion Test

The coated bars are submerged in a vessel containing tap water at 75°C and removed at specified times. After cooling for 45 minutes, an X-cut is made through the coating to the steel. The adhesion rating of the coating is ascertained by the ease with which the coating is removed with a tile knife at the X-cut. A rating of 1 indicates easy, complete removal of the coating from the steel bar. A rating of 4 indicates that the coating was removed with extreme difficulty.

Hot Water Immersion Test

75°C tap water, 216°C coating temperature; Immersion Time

| Ex. 1 | 1 day | 2 days | 4 days | 5 days | 8 days | 11 days | 14 days |
|---|---|---|---|---|---|---|---|
| 1 | 1 | - | - | - | - | - | - |
| 2 | 3 | - | 3 | - | 1-2 | - | - |
| 3 | 3 | - | 3 | - | 3 | 2 | 1-2 |
| 4 | 3 | - | 2 | - | 1 | - | - |
| 5 | 2 | - | 2 | - | 1-2 | 1-2 | 1-2 |
| 6 | 3 | - | 3-4 | - | 2-3 | 2-3 | 2 |
| 7 | 1 | - | - | - | - | - | - |
| 8 | 3 | - | 2 | - | 1-2 | 1 | - |
| 9 | 3 | - | 2 | - | 1 | - | - |
| 10 | 4 | - | 4 | - | 4 | 3 | 2 |
| 11 | - | 1-2 | - | 1-2 | 1-2 | - | - |
| 12 | - | 2-3 | - | 2 | 1-2 | - | - |
| 13 | - | 4 | - | 4 | 3 | - | - |
| 14 | - | 4 | - | 4 | 4 | - | - |
| 15 | - | 4 | - | 4 | 4 | - | - |
| 16 | - | 4 | - | 4 | 4 | - | - |
| 17 | - | 1 | - | - | - | - | - |
| 18 | - | - | 4 | - | - | - | - |
| 19 | 4 | - | - | - | - | 4 | - |
| 20 | - | - | - | 4 | - | 4 | - |
| 21 | - | - | - | 4 | - | 4 | - |
| 22 | 1 | - | - | - | - | - | - |
| 23 | 4 | - | - | - | - | - | - |

Adhesion Rating:

1 - coating easily removed

2 - coating can be removed

3 - coating removed with difficulty

4 - coating removed with extreme difficulty

Hot Water Immersion Test (continued)

75° C tap water

Coating Temp/Time of Immersion

| Example | 149° C | | 177° C | | 241° C | |
|---|---|---|---|---|---|---|
| | 1 day | 3 days | 1 day | 3 days | 1 day | 3 days |
| 16 | 1-2 | 1-2 | 3-4 | 3 | 4 | 3 |
| 17 | 1 | 1 | 2 | 2 | 2 | 1-2 |

Cathodic Disbondment Test

Blast-cleaned steel panels (5" x 5") are heated at 204° C and coated to a thickness of 350-410 um using electrostatic spray techniques. A hole is drilled through the coating (3.2 mm diameter), an electrical current is applied to the steel panel (electrolyte = 3% NaCl in distilled water; 3 volts), and the panel is then placed in an oven at 66° C. The coating disbondment diameter at the drilled hole is determined after 9 days by flaking away with a tile knife the easily removed portion of the coating.

Cathodic Disbondment Test

| Example | Disbondment Diameter (duplicate tests) (mm) |
|---|---|
| 15 | 16, 19 |
| 17 | 38, 44 |

Discussion

The use of aromatic compounds having adjacent hydroxyls or hydroxyls in available adjacent positions improves the hot/wet adhesion of various epoxy polymer coatings to steel. Specifically these polyols exhibit the improved adhesion in amine/epoxy systems (ex. 2-8, 12-16) relative to a control (ex. 1,17), a phenol/epoxy system (ex. 10) relative to the control (ex. 9), and in a carboxyl/epoxy systems (ex. 12, 23) relative to controls (ex. 11, 22). Epoxy resins of varying epoxy equivalent weight can be used as evidenced by ex. 5 (E.E.W. = 730-840) ex. 2 (E.E.W. = 875-975) and ex. 6 (E.E.W. = 1,600-2,000).

Many different examples of formulations incorporating the aromatic polyols of this application have been included. These range from the very simple monomeric compounds (catechol, pyrogallol; ex. 6,12) to polynuclear polyhydroxy compounds (ex. 2,3,5,10, 18, 19, 20) to bifunctional compounds (ex. 4, 8).

As a test of our assertion that the improved hot/wet adhesion is due to the positioning of the hydroxyl groups, a formulation using a polyhydroxy phenol containing non-adjacent hydroxyls is tried (ex. 7; 1,3,5-trihydroxybenzene) and found not to give any improved adhesion in the hot water immersion test.

Several different concentrations of pyrogallol are studied (ex. 13-16). Over the range studied (0.1 to 1.0% relative to epoxy by weight), there is only a very small drop in adhesion in the hot water immersion test.

Another example of the versatility of this invention involves the coating of steel substrates at varying temperatures. Improved adhesion is seen at relatively low coating temperatures (149° C, 177° C and 216° C) in the hot water immersion test.

Finally, a complementary test, the cathodic disbondment test, confirms the improved adhesion of compositions of this application.

The principles, preferred embodiments and modes of operation of the present invention have been described in the foregoing specification. The invention which is intended to be protected herein, however, is not to be construed as limited to the particular forms disclosed, since these are to be regarded as illustrative rather than restrictive. Variations and changes may be made by those skilled in the art without departing from the spirit of the invention.

## Claims

1. A curable powdered coating composition comprising

a) at least one uncured epoxy resin having an epoxy equivalent weight greater than 99, and

b) one or more aromatic compounds containing at least one of the following moieties

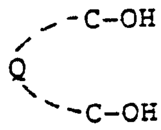 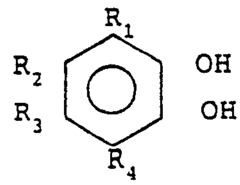 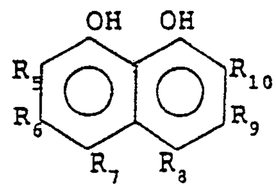

wherein Q comprises any combination of carbon, oxygen, nitrogen and/or sulfur atoms necessary to complete an aromatic ring and $R_1$-$R_{10}$ are independently a hydrogen or a substitution group.

2. A composition according to Claim 1 containing at least 0.05 parts by weight of the aromatic compound.

3. A composition according to either of Claims 1 and 2, further comprising

c) an epoxy resin catalyst.

4. A composition according to Claim 3 wherein the catalyst is selected from 2-methylimidazole adducts, 2,4,6-tris(dimethylaminomethyl)phenol, N, N- dimethylbenzylamine and stannous octoate.

5. A composition according to any one of Claims 1 to 4 wherein adjacent substitution groups form fused aromatic, nonaromatic or heterocyclic rings of up to 7 ring atoms.

6. A composition according to Claim 1 wherein the aromatic compound contains at least one moiety selected from the group consisting of catechol, pyrogallol, 2,3-dihydroxypyridine, o-dihydroxyanthraquinone, dithranol, o-dihydroxynaphthalene, and 1,8-dihydroxynaphthalene.

7. A composition according to any one of Claims 1 to 6 wherein the epoxy resin is selected from bisphenol A or bisphenol F epoxy resin, triglycidyl isocyanurate, and epoxidized novolak.

8. A composition according to any one of Claims 1 to 7 further comprising a curing agent.

9. A method for providing an anti-corrosive coating on a metal substrate comprising applying the powdered coating composition of any one of Claims 1 to 8 to the substrate and heating to coalesce and cure said composition.

10. A method according to Claim 9 wherein the powdered coating composition is applied by fluid bed technique.

11. A method according to Claim 9 wherein the powdered coating composition is applied by powder spray coating.

12. A method according to any one of Claims 8 to 11 wherein the substrate is preheated.

13. A method according to any one of Claims 8 to 12 wherein the application method is an electrostatic method.

14. A method according to any one of Claims 8 to 13 wherein the substrate is steel.